## Europäisches Patentamt
## European Patent Office
## Office européen des brevets

(19)

(11) Veröffentlichungsnummer: **0 103 241**
**B1**

(12)

# EUROPÄISCHE PATENTSCHRIFT

(45) Veröffentlichungstag der Patentschrift:
**04.12.85**

(51) Int. Cl.⁴: **C 07 C 37/16,** C 07 C 39/06

(21) Anmeldenummer: **83108648.3**

(22) Anmeldetag: **02.09.83**

(54) **Verfahren zur Herstellung von o-Benzylphenol.**

(30) Priorität: **14.09.82 DE 3234036**

(43) Veröffentlichungstag der Anmeldung:
**21.03.84 Patentblatt 84/12**

(45) Bekanntmachung des Hinweises auf die Patenterteilung:
**04.12.85 Patentblatt 85/49**

(84) Benannte Vertragsstaaten:
**DE FR GB NL**

(56) Entgegenhaltungen:
**DE - A - 2 237 591**
**US - A - 2 448 942**
**US - A - 4 105 699**

(73) Patentinhaber: **BAYER AG, Konzernverwaltung RP Patentabteilung, D-5090 Leverkusen 1 Bayerwerk (DE)**

(72) Erfinder: **Hemmerich, Heinz-Peter, Dr., Sebastian Knelpp Weg 11, D-4150 Krefeld (DE)**
Erfinder: **Schulte-Huermann, Werner, Dr., Helmut Macke Strasse 10, D-4150 Krefeld (DE)**
Erfinder: **Dohm, Heinz, Dr., Heyenbaumstrasse 86 b, D-4150 Krefeld (DE)**

**Beschreibung**

Die Erfindung betrifft ein Verfahren zur Herstellung von o-Benzylphenol durch Umsetzung von Phenol mit Dibenzylether in Gegenwart von Kondensationsmitteln.

Aus der deutschen Reichspatentanmeldung 81 666 (bekanntgemacht am 05. 11. 1942) ist ein Verfahren zur Herstellung von Aralkylphenolen bekannt, das dadurch gekennzeichnet ist, daß man Phenole oder ihre Substitutionsprodukte, die eine freie p- und/oder o-Stellung enthalten, mit Aralkylethern, genannt ist auch Dibenzylether, in Gegenwart katalytisch-wirkender Kondensationsmittel erhitzt. Als Kondensationsmittel werden genannt: Aluminiumchlorid, Zinkchlorid, Silicagel und Bleicherde.

Nachteilig bei diesem Verfahren ist insbesondere, daß es nicht selektiv verläuft, was bedeutet, daß neben dem o-Benzylphenol zu etwa gleichen Teilen auch das p-Benzylphenol gebildet wird (siehe Beispiel 2 der Patentanmeldung 81 666). Zur Reindarstellung von o-Benzlphenol muß daher das unerwünschte p-Benzylphenol in technisch aufwendiger Weise abgetrennt werden.

Weiterhin ist ein Verfahren zur gezielten Herstellung von o-Benzylphenol aus der US-PS 4 105 699 bekannt. Nach dem dort beschriebenen Verfahren wird Benzylalkohol und Phenol in Gegenwart von aktivierter Tonerde mit einer $\alpha$-Aluminiumoxidmonohydrat-Kristallstruktur als Katalysator erhitzt, wobei o-Benzylphenol praktisch frei vom p- und m-Isomeren erhalten wird. Dieses Verfahren besitzt jedoch den Nachteil, daß von dem teuren Benzylalkohol ausgegangen wird und die o-Benzylphenol-Ausbeute sowie der Phenolumsatz für ein großtechnisches Verfahren unbefriedigend sind. Dadurch gestaltet sich das in der US-PS 4 105 699 beschriebene Verfahren wenig wirtschaftlich.

Die Erfindung betrifft ein Verfahren zur Herstellung von o-Benzylphenol durch Umsetzung von Phenol mit Dibenzylether in Gegenwart von Kondensationsmitteln bei erhöhter Temperatur, dadurch gekennzeichnet, daß man die Umsetzung von Phenol mit Dibenzylether in einem Molverhältnis von 1 : 0,05 bis 1 : 0,7 (Phenol zu Dibenzylether) bei Temperaturen von 100−300°C durchführt, wobei als Kondensationsmittel $\gamma$-Aluminiumoxid in einer Menge von 1−50 Gew.-%, bezogen auf eingesetztes Phenol, eingesetzt wird.

Das in das erfindungsgemäße Verfahren eingesetzte $\gamma$-Aluminiumoxid (s. Gmelins Handbuch der Anorganischen Chemie, 8. Aufl., Aluminium, Teil B, S. 78 ff., Verlag Chemie, Berlin, 1934) besitzt eine spezifische Oberfläche (BET-Oberfläche) von etwa 100 bis 350 m$^2$/g, bevorzugt 150 bis 300 m$^2$/g. Der Al$_2$O$_3$-Gehalt beträgt etwa 80 bis 99 Gew.-%, bevorzugt 90 bis 98 Gew.-%. Das Schüttgewicht beträgt etwa 600 bis 1100 g/l, bevorzugt 750 bis 900 g/l.

Die Menge des erfindungsgemäß eingesetzten Kondensationsmittels beträgt bevorzugt 3 bis 20 Gew.-%, bezogen auf eingesetztes Phenol.

Phenol und Dibenzylether werden bevorzugt in einem Molverhältnis von 1 : 0,45 bis 1 : 0,1 (Phenol zu Dibenzylether) in das erfindungsgemäße Verfahren eingesetzt.

Die Umsetzung erfolgt dabei vorzugsweise im Temperaturbereich von 160 bis 190°C, wobei bei Normaldruck gearbeitet wird.

Das Reaktionswasser kann zusammen mit wenig Phenol ausgetragen werden. Man setzt dann mit Vorteil etwas mehr Phenol in die Reaktion ein, ohne dabei die obengenannten Molverhältnisse von Phenol zu Dibenzylether zu überschreiten. Zur Entfernung des Reaktionswassers ist es auch möglich, eine geeignete Menge von beispielsweise Toluol als Azeotropbildner dem Reaktionsgemisch zuzusetzen. Weitere geeignete Azeotropbildner sind z. B. Benzol, Xylol, Heptan, Octan, Cyclohexan, Perchlorethylen, Chlorbenzol und/oder Nitroethan. Die benötigte Menge an Azeotropbildner hängt vornehmlich von den geometrischen Abmessungen des Wasserabscheiders ab. Als Richtgröße können im allgemeinen ca. 3 bis 20 Gew.-%, bevorzugt 5 bis 15 Gew.-%, bezogen auf eingesetztes Phenol und eingesetzten Dibenzylether, angesehen werden.

Die Reaktionszeit hängt im wesentlichen von der Aktivität des Kondensationsmittels sowie dessen mengenmäßigem Einsatz ab und beträgt etwa 3 bis 50 Stunden, bevorzugt 5 bis 20 Stunden.

Nach Beendigung der Reaktion kann das Reaktionsgemisch gegebenenfalls nach Abtrennen des Katalysators destillativ durch z. B. Vakuumdestillation aufgearbeitet werden. Man erhält das o-Benzylphenol nach dem erfindungsgemäßen Verfahren in einer Ausbeute von ca. 85% der Theorie, bezogen auf Phenol, bzw. von ca. 90% der Theorie, bezogen auf Dibenzylether. Die Reinheit des erhaltenen o-Benzylphenols ist außerordentlich hoch und beträgt mindestens 99%.

Das erfindungsgemäße Verfahren kann beispielsweise wie folgt durchgeführt werden: In einer Rühraparatur mit Rückflußkühler und Wasserabscheider werden geschmolzenes Phenol, der Dibenzylether sowie das $\gamma$-Aluminiumoxid vorgelegt. Ferner wird eine geeignete Menge Toluol als Azeotropbildner dem Reaktionsgemisch hinzugefügt. Man heizt unter Rühren das Reaktionsgemisch auf, bis sich ein milder Rückfluß eingestellt hat. Zu Beginn der Reaktion beträgt die Reaktionstemperatur etwa 170°C, zum Ende der Umsetzung werden ca. 190°C erreicht. Die Reaktion wird fortgesetzt, bis annähernd die theoretische Menge Reaktionswasser mit dem Toluol ausgeschleust ist. Zweckmäßigerweise wird der Fortgang der Umsetzung durch z. B. Gaschromatographie verfolgt. Nach Abtrennung des Katalysators durch z. B. Dekantieren oder Filtrieren wird das Reaktionsgemisch unter Vakuum destilliert, wobei man das o-Benzylphenol in sehr hoher Reinheit erhält.

Es ist auch möglich, die Vakuumdestillation direkt, d. h. ohne vorherige Entfernung des Katalysators, nach Beendigung der Reaktion durchzuführen.

Das erfindungsgemäße Verfahren kann sowohl kontinuierlich als auch diskontinuierlich durchgeführt werden.

Die Vorteile des erfindungsgemäßen Verfahrens sind im Vergleich zum Stand der Technik insbesondere in den hohen Ausbeuten und der außerordentlichen Reinheit des erhaltenen o-Benzylphenols zu sehen. Dabei ist noch die besonders wirtschaftliche Arbeitsweise des erfindungsgemäßen Verfahrens zu berücksichtigen.

Es überrascht besonders, daß gegenüber der deutschen Reichspatentanmeldung 81 666, ausgehend vom Phenol und Dibenzylether o-Benzylphenol mit einer solch hohen Selektivität erhalten wurde und sich die Ausbeute an o-Benzylphenol, verglichen mit der nach der US-PS 4 105 699 erhaltenen Ausbeute, noch beträchtlich steigern ließ.

o-Benzylphenol dient unter anderem als solches bzw. nach Chlorierung in Form der Chlorbenzylphenole als mikrobizides Mittel (siehe z. B.: Dr. K. H. Wallhäußer, Prof. Dr. Dr. H. Schmidt in: »Sterilisation, Desinfektion, Konservierung, Chemotherapie«, Georg Thieme Verlag, Stuttgart, 1967, S. 183/184).

### Beispiel 1

In einer Rührapparatur mit Rückflußkühler und Wasserabscheider wurden 564,7 g (6 Mol) Phenol, 356,9 g (1,8 Mol) Dibenzylether sowie 45,2 g γ-Aluminiumoxid mit einer BET-Oberfläche von ca. 240 m$^2$/g, einem Glühverlust von ca. 5 Gew.-% und einem Al$_2$O$_3$-Gehalt von ca. 95 Gew.-% vorgelegt (Schüttgewicht ca. 860 g/l). Nach Zufügen von 100 g Toluol wurden innerhalb 10 Stunden bei Rückflußtemperatur (180 bis 190°C) etwa 26 g Wasser ausgeschleust. Nach dem Abtrennen des Aluminiumoxids wurden 890 g eines Rohproduktes erhalten, dessen Zusammensetzung gaschromatographisch wie folgt bestimmt wurde: 23% Phenol, 0,2% Dibenzylether, 67% o-Benzylphenol, 0,1% p-Benzylphenol, 7% Dibenzylphenol.

Die aus dem Gaschromatogramm des Rohprodukts errechenbaren o-Benzylphenolausbeuten betragen 84% der Theorie, bezogen auf Phenol und 89% der Theorie, bezogen auf Dibenzylether. Der Phenolumsatz beläuft sich auf 64%.

Bei der anschließenden destillativen Aufarbeitung im Vakuum wurden 190 g Phenol zurückgewonnen. Nach einem Zwischenlauf von ca. 60 g destillierten 530 g eines ca. 99%igen o-Benzylphenols über. Unter Berücksichtigung des im Zwischenlauf enthaltenen o-Benzylphenols berechnen sich die Ausbeuten nach der Destillation für o-Benzylphenol auf 81% der Theorie, bezogen auf Phenol, und auf 86% der Theorie, bezogen auf Dibenzylether.

### Beispiel 2

In einer Rührapparatur mit Rückflußkühler und Wasserabscheider wurden 188,2 g (2 Mol) Phenol, 99,1 g (0,5 Mol) Dibenzylether sowie 19 g γ-Aluminiumoxid mit einer BET-Oberfläche von ca. 150 m$^2$/g, einem Glühverlust von ca. 4 Gew.-% und einem Al$_2$O$_3$-Gehalt von ca. 94,5 Gew.-% vorgelegt (Schüttgewicht ca. 850 g/l). Nach dem Zufügen von 50 g Toluol wurden innerhalb von 15 Stunden bei Rückflußtemperatur (180 bis 190°C) etwa 8 g Wasser ausgeschleust. Nach Abtrennung des Katalysators wurden 273 g eines Rohproduktes erhalten, dessen Zusammensetzung gaschromatographisch wie folgt bestimmt wurde: 32% Phenol, 0,1% Dibenzylether, 62% o-Benzylphenol, weniger als 0,1% p-Benzylphenol, 5% Dibenzylphenol. Die hieraus errechneten o-Benzylphenolausbeuten betragen: 86% der Theorie, bezogen auf Phenol und 92% der Theorie, bezogen auf Dibenzylether. Der Phenolumsatz beträgt hierbei 54%.

### Beispiel 3

In einer Destillationsapparatur mit Rührer wurden 1930 g (20,5 Mol) Phenol, 1190 g Dibenzylether (6 Mol) sowie 193 g γ-Aluminiumoxid mit einer BET-Oberfläche von ca. 250 m$^2$/g, einem Glühverlust von ca. 5 Gew.-% und einem Al$_2$O$_3$-Gehalt von ca. 94,5 Gew.-% vorgelegt und unter Normaldruck auf Rückflußtemperatur aufgeheizt. Bei ansteigender Temperatur im Reaktionssumpf (180 bis 210°C) ließ sich das entstehende Reaktionswasser azeotrop mit Phenol innerhalb von 4 Stunden abdestillieren. Nach Abtrennung des Katalysators wurden 3170 g eines Rohproduktes erhalten, dessen Zusammensetzung gaschromatographisch wie folgt bestimmt wurde: 31% Phenol, 0,3% Dibenzylether, 62% o-Benzylphenol, 0,2% p-Benzylphenol, 5,5% Dibenzylphenol. Die hieraus berechneten o-Benzylphenolausbeuten betragen: 93% d.Th., bezogen auf Phenol und 88% d.Th., bezogen auf Dibenzylether. Der Phenolumsatz beträgt hierbei 56%.

### Patentansprüche

1. Verfahren zur Herstellung von o-Benzylphenol durch Umsetzung von Phenol mit Dibenzylether in Gegenwart von Kondensationsmitteln bei erhöhter Temperatur, dadurch gekennzeichnet, daß man die Umsetzung von Phenol mit Dibenzylether in einem Molverhältnis von 1 : 0,05 bis 1 : 0,7 (Phenol zu Dibenzylether) bei Temperaturen von 100—300°C durchführt, wobei als Kondensationsmittel γ-Aluminiumoxid in einer Menge von 1—50 Gew.-%, bezogen auf eingesetztes Phenol, eingesetzt wird.

2. Verfahren nach Anspruch 1, dadurch gekennzeichnet, daß man das Kondensationsmittel in einer Menge von 3 bis 20 Gew.-%, bezogen auf eingesetztes Phenol, einsetzt.

3. Verfahren nach Ansprüchen 1 und 2, dadurch gekennzeichnet, daß das $\gamma$-Aluminiumoxid eine spezifische Oberfläche von 100 bis 350 m$^2$/g und einen Al$_2$O$_3$-Gehalt von 80 bis 99 Gew.-% besitzt.

4. Verfahren nach Ansprüchen 1 bis 3, dadurch gekennzeichnet, daß das $\gamma$-Aluminiumoxid eine spezifische Oberfläche von 150 bis 300 m$^2$/g und einen Al$_2$O$_3$-Gehalt von 90 bis 98 Gew.-% besitzt.

## Claims

1. Process for the preparation of o-benzylphenol by reacting phenol with dibenzyl ether in the presence of condensation agents at an elevated temperature, characterised in that the reaction of phenol with dibenzyl ether is carried out in a molar ratio of 1 : 0.05 to 1 : 0.7 (phenol : dibenzyl ether) at temperatures of 100—300°C, $\gamma$-aluminium oxide being used as the condensation agent in a quantity of 1—50% by weight, relative to phenol used.

2. Process according to Claim 1, characterised in that the condensation agent is used in a quantity of 3 to 20% by weight, relative to phenol used.

3. Process according to Claims 1 and 2, characterised in that the $\gamma$-aluminium oxide has a specific surface area of 100 to 350 m$^2$/g and an Al$_2$O$_3$ content of 80 to 99% by weight.

4. Process according to Claims 1 to 3, characterised in that the $\gamma$-aluminium oxide has a specific surface area of 150 to 300 m$^2$/g and an Al$_2$O$_3$ content of 90 to 98% by weight.

## Revendications

1. Procédé de production de o-benzylphénol par réaction de phénol avec l'éther de dibenzyle en présence d'agents de condensation à température élevée, caractérisé en ce qu'on conduit la réaction du phénol avec l'éther de dibenzyle dans un rapport molaire (phénol : éther de dibenzyle) de 1 : 0,05 à 1 : 0,7 à des températures de 100 à 300°C, en utilisant alors comme agent de condensation de l'oxyde d'aluminium $\gamma$ en une quantité de 1 à 50% en poids par rapport au phénol utilisé.

2. Procédé suivant la revendication 1, caractérisé en ce qu'on utilise l'agent de condensation en une quantité de 3 à 20% en poids par rapport au phénol utilisé.

3. Procédé suivant les revendications 1 et 2, caractérisé en ce que l'oxyde d'aluminium $\gamma$ a une surface spécifique de 100 à 350 m$^2$/g et une teneur en Al$_2$O$_3$ de 80 à 99% en poids.

4. Procédé suivant les revendications 1 à 3, caractérisé en ce que l'oxyde d'aluminium $\gamma$ a une surface spécifique de 150 à 300 m$^2$/g et une teneur en Al$_2$O$_3$ de 90 à 98% en poids.